# EUROPEAN PATENT SPECIFICATION

(11) **EP 2 976 075 B1**
(45) Date of publication and mention of the grant of the patent: **17.07.2019**
(21) Application number: 14718921.1
(22) Date of filing: 19.03.2014
(51) Int. Cl.: A61K 31/357, A61P 3/08, A61P 5/48

(54) **SILYBIN FOR THE TREATMENT OF METABOLIC OXIDATIVE ALTERATIONS IN PATIENTS WITH NON-ALCOHOLIC STEATOHEPATITIS (NASH)**
SILYBIN ZUR BEHANDLUNG VON METABOLISCHEN OXIDATIVEN ÄNDERUNGEN IN PATIENTEN MIT NON-ALKOHOLISCHEN STEATOHEPATITIS (NASH)
SILIBYN POUR TRAITER ALTERATIONS OXIDATIVES METABOLIQUES DANS DES PATIENTS AVEC STEATOHEPATITE NON-ALCOHOLIQUE (NASH)

(30) Priority: 20.03.2013 IT MI20130425
(43) Date of publication of application: 27.01.2016
(73) Proprietor: ISTITUTO BIOCHIMICO ITALIANO GIOVANNI LORENZINI S.p.A., 04011 Aprilia (IT)
(72) Inventor: LOGUERCIO, Carmelina, I-80139 Napoli (IT); FEDERICO, Alessandro, I-80128 Napoli (IT); STIUSO, Paola, I-80137 Napoli (IT)
(74) Representative: Cattaneo, Elisabetta
(86) International application number: PCT/EP2014/055499
(87) International publication number: WO 2014/147119

(56) References cited:
- WO-A2-2013/086323
- CN-A- 1 513 444
- DE-A1-102004 054 133
- US-A1- 2010 239 552
- US-A1- 2010 255 127
- SATAPATHY S K ET AL: "Novel treatment modalities for nonalcoholic steatohepatitis", TRENDS IN ENDOCRINOLOGY AND METABOLISM, ELSEVIER SCIENCE PUBLISHING, NEW YORK, NY, US, vol. 21, no. 11, 1 November 2010 (2010-11-01), pages 668-675, XP027446389, ISSN: 1043-2760, DOI: 10.1016/J.TEM.2010.08.003 [retrieved on 2010-09-27]
- FEDERICO ALESSANDRO ET AL: "The effect of a chronic treatment with silibinin on insulin-resistance and plasma markers of liver fibrosis in patients with chronic liver damage.", HEPATOLOGY, vol. 40, no. 4, Suppl. 1, October 2004 (2004-10), page 583A, XP008165785, & 55TH ANNUAL MEETING OF THE AMERICAN-ASSOCIATION-FOR-THE-STUDY-OF-LIVE R-DISEASES (AASLD); BOSTON, MA, USA; OCTOBER 29 -NOVEMBER 02, 2004 ISSN: 0270-9139
- MALAGUARNERA M ET AL: "Myocardial oxidative stress in an experimental model of nonalcoholic fatty liver disease: role of silibinin", FEBS JOURNAL, WILEY-BLACKWELL PUBLISHING LTD, GB, [Online] vol. 278, no. Supplement s1, 1 June 2011 (2011-06-01), page 465, XP008165777, ISSN: 1742-464X, DOI: 10.1111/J.1742-4658.2011.08138.X Retrieved from the Internet: URL:http://onlinelibrary.wiley.com/doi/10. 1111/j.1742-4658.2011.08138.x/pdf> [retrieved on 2011-07-01]
- SALAMONE FEDERICO ET AL: "Silibinin modulates lipid homeostasis and inhibits nuclear factor kappa B activation in experimental nonalcoholic steatohepatitis", TRANSLATIONAL RESEARCH, ELSEVIER, AMSTERDAM, NL, [Online] vol. 159, no. 6, 1 June 2012 (2012-06-01), pages 477-486, XP008165776, ISSN: 1931-5244, DOI: 10.1016/J.TRSL.2011.12.003 Retrieved from the Internet: URL:http://ac.els-cdn.com/S193152441100430 0/1-s2.0-S1931524411004300-main.pdf?_tid=e 79e1ea0-486f-11e3-9c9e-00000aacb361&acdnat =1383913301_c13a6082d9d4a2710343785513e37e cc>
- MATTEUCCI ELENA ET AL: "Liver Disease in Diabetes Mellitus: Potential Therapeutic Value of Vitamin E-Silybin Phytosomal Complex", IMMUNOLOGY, ENDOCRINE AND METABOLIC AGENTS IN MEDICINAL CHEMISTRY, BENTHAM SCIENCE PUBLISHERS LTD, NL, vol. 10, no. 2, 1 June 2010 (2010-06-01), pages 84-90, XP009170635, ISSN: 1871-5222

## Description

### FIELD OF THE INVENTION

The present invention relates to a composition comprising silybin for the treatment of metabolic oxidative alterations in non-alcoholic steatohepatitis (NASH).

### BACKGROUND OF THE ART

Oxidative stress, lipid peroxidation, antioxidant therapy, these terms are very common in the literature and in everyday clinical practice in recent years. In fact, in the pathogenesis of various diseases, including cancer, considerable emphasis is often given to the formation of free radicals and their entrapment by antioxidant substances.

In humans, evidence has clearly been provided that an increase in circulating oxidative damage markers is an expression of organ pathology.

Among the oxidative stress markers considered expression of pathology of various organs and systems, the increase of products from lipid peroxidation plays a prominent role. Lipid peroxidation markers in the circulation are present in many pathological conditions as an expression of peroxidation of cell membranes. Among various diseases, surely the metabolic syndrome, but also the chronic liver disease and the advanced stages of many cancers, represent pathognomonic situations of chronic diseases, in which the oxidative stress plays a key role. Non-alcoholic steatohepatitis (NASH) is the hepatic manifestation of metabolic syndrome. Non-alcoholic steatohepatitis (NASH) is a type of chronic damage to the liver belonging to metabolic diseases and represents the degenerative inflammatory progression of the non-alcoholic fatty liver disease (NAFLD).

The presence of the metabolic syndrome or of one or of more components of this syndrome (i. e. visceral obesity, hypertension, hypertriglyceridemia, fasting hyperglycemia) greatly increases the risk of progression of non-alcoholic hepatic steatosis to steatohepatitis (NASH). NASH is in fact almost always present in patients with metabolic syndrome and affects their morbidity and mortality.

It is established through several steps which involve the modulation of a large number of genes and proteins, starting from an altered trafficking of lipids between the periphery (adipose tissue) and liver, with final accumulation of fatty acids in the liver, peroxidation of both circulating and intrahepatic lipids, which induce, through oxidative stress, hepatocyte inflammation and death, up to triggering of reparative phenomena, such as fibrosis and cirrhosis. At present, no pharmacological agents have been approved for the treatment of metabolic syndrome, in particular for the treatment of NASH. In the latter case, a long-term treatment was attempted with insulin sensitizing agents or with high doses of vitamin E, without definitive results.

While the use of natural products is increasing throughout the world, the fact that their use is not always safe and effective is, however, also known. Patients with non-alcoholic fatty liver disease (NAFLD) have been shown to respond positively when subjected to chronic administration of silybin. In particular, treatment of patients with NAFLD with silybin has been shown to improve the levels of transaminases, insulin resistance and liver histology. NASH is characterized, histologically, by a chronic inflammation and early fibrosis, as compared to other chronic liver diseases included in NAFLD.

Object of the present invention is to provide a treatment of the metabolic syndrome, when associated to NASH

### SUMMARY OF THE INVENTION

The inventors of the present invention have identified among the patients with metabolic syndrome, when associated to NASH, a group of patients presenting a lipidomic profile alteration associated with high values of TBARS in serum, and have surprisingly discovered that treatment with a composition including silybin allows normalization of said lipidomic profile, thus having effects in the treatment of non-alcoholic steatohepatitis.

The invention thus relates to a composition for use in the treatment of metabolic oxidative alterations in patients with non-alcoholic steatohepatitis (NASH), wherein said composition comprises silybin, and wherein the treatment is administered to patients having an altered lipidomic profile according to the claims.

In the present invention, when the definition "altered lipidomic profile" is used, it is meant the lipidomic profile of a patient with metabolic syndrome, preferably also with NASH, who is positive to the TBARS assay, who specifically show high values of TBARS in the TBARS assay ((2-ThioBarbituric Acid Reactive Substances (TBARS)). The TBARS assay measures in a serum sample of a patient the TBARS, i.e. the by-products generated by a process of lipid peroxidation, through the use of barbituric acid (Armstrong, D., and Browne, R. The analysis of free radicals, lipid peroxides, antioxidant enzymes and compounds to oxidative stress as applied to the clinical chemistry laboratory. Free Radicals in Diagnostic Medicine 366, 43-58 1994). In the present invention, when it is stated that high levels of TBARS or low levels of TBARS are present, it is meant that the values found in the TBARS assay are higher or lower than those observed in healthy subjects. In the present invention, while referring to average values of TBARS in healthy subjects average values of approximately 0.01±0.002 µM/µgprot (µM of TBARS with respect to µg of plasma proteins) are meant.

The inventors of the present invention have therefore identified a specific group of patients with the metabolic syndrome and with also NASH, who have an alteration of serum lipidomics and who are responsive to treatment with a composition comprising silybin. The group of patients identified by the inventors in the group of patients with metabolic syndrome with also NASH, have a lipidomic profile associated with altered lipid peroxidation (high values of TBARS) that distinguishes it from the group of patients with metabolic syndrome with also NASH, who, in basic conditions, do not show high levels of circulating lipid peroxidation markers (TBARS). The two groups of patients are in no way overlapping on the basis of the values of TBARS determined by the TBARS assay, as will be evident from the experimental section below. Upon completion of the treatment with the composition including silybin, the group of patients of the invention showed a normalization of the serum lipidomic profile, with a corresponding lowering of the values of TBARS in the TBARS assay, confirming the treatment of oxidative stress typical of NASH pathology.

The invention will now be described in detail and with reference to the following figures.

### DESCRIPTION OF THE FIGURES

Figure 1 includes graphs of mass spectroscopy of a pool of serum samples of the control group (CTR) (a)), the first group of NASH patients at time zero (TO, Group 1) (b)) and the second group of NASH patients at time zero (TO, Gruppo 2) (c)).
Figure 2 includes the graphs of mass spectroscopy of a pool of serum samples of the first group of NASH patients after 12 months of treatment (T12) with the composition of the invention (Group 1) (b)) and the second group of NASH patients after 12 months of treatment (T12) with the composition of the invention (Group 2) (c)).

### DETAILED DESCRIPTION OF THE INVENTION

The invention therefore relates to a composition for use in the treatment of metabolic oxidative alterations in non-alcoholic steatohepatitis (NASH), and wherein said composition comprises silybin, and wherein the treatment is administered to patients having an altered lipidomic profile characterised by TBARS value higher than healthy subjects as defined in the claims.

The group of patients having the "altered lipid profile" show an high TBARS value in TBARS assay with respect to healthy subjects. The TBARS assay detects the substances reactive to barbituric acid, which are formed as by-products of lipid peroxidation. Therefore, TBARS levels represent a marker of lipid peroxidation. A patient having the metabolic syndrome with also NASH, who is positive to the TBARS assay, show high values of TBARS in the TBARS assay, i.e. a large number of lipidic peroxidation by-products of the cell membranes is present in the serum of the patient.

The inventors of the present invention have therefore identified a sub-group of patients with the metabolic syndrome with also NASH, who have an alteration of lipidomics and who are responsive to treatment with a composition comprising silybin.

The composition of the invention comprises silybin, preferably complexed with phosphatidylcholine. Silybin used in the invention can be in the form of an extract of *Silybum marianum,* i.e. Milk thistle. It is known that extracts of Milk thistle can be titrated with silybin or silymarin, in the invention all these extracts can be used. Extracts of Milk thistle are preferred, titrated with silybin in the range of 20 to 90%, more preferably of 30 to 70%. Even more preferably, the composition of the invention comprises an extract of *Silibum marianum* titrated with 33% silybin.

According to the invention, the composition comprises preferably vitamin E.

Advantageously, the composition of the invention includes an extract of Milk thistle, complexed with phosphatidylcholine and vitamin E acetate.

The composition of the invention comprises also excipients suitable for the preparation of a pharmaceutical formulation.

Preferably, according to the invention, the daily dose of silybin is in the range of 150 to 300 mg/day, being even more preferable a dose of about 200 mg/day of silybin.

The group of patients with the metabolic syndrome with also NASH, and identified in the treatment according to the invention have therefore an altered lipidomic profile, which are normalized with the composition of the invention, as will be evident from the experimental part that follows.

### Example 1

### Preparation of the composition used in the invention

Silybin in the form of an extract of Milk thistle complexed with phosphatidylcholine, was added to vitamin E acetate in the amounts indicated in Table 1:

**Table 1**

| Composition | mg |
|---|---|
| Saccharin | 2491.36 |
| Carrageenan | 60.00 |
| Maltodextrin | 200.00 |
| Extract of Milk thistle complexed with phosphatidylcholine (silybin 47.52 mg and phosphatidylcholine 96.48 mg) | 144.00 |
| Vitamin E acetate 50% | 44.64* |
| Flavours | 60.00 |
| Total Weight | 3000.00 |

| | |
|---|---|
| * equal to 15 mg Vit E | |

The composition so obtained was administered to the patients in the form of sachets of powder composition.

### Example 2:

### Identification of the two groups of patients

We enrolled 20 patients affected by metabolic syndrome and non-alcoholic steatohepatitis (NASH). We also enrolled 20 healthy subjects, who formed the control group. The serum of 20 patients was assessed at basal conditions and after 12 months of treatment with the composition of the invention, by performing the TBARS assay. Then, the level of TBARS, i.e. the level of reactive substances as lipidic peroxidation by-products of the cell membranes in the serum of all 40 subjects was evaluated.

The analysis performed on the serum of these patients showed the presence of two distinct groups of patients (Tables 2 and 3). 11 of the 20 patients involved in the study were in group 1 and 9 belonged to group 2, based on the values of TBARS found and reported in Table 2 already divided into two groups. Table 2 also shows the values of TBARS measured in patients not only at time zero (TO), but even after 12 months of treatment (T12).

**Table 2. Values of TBARS (µM/µgprot.) measured in the serum of patients with metabolic syndrome and NASH belonging to group 1 (n=11) and of group 2 (n=9). TBARS = substances reactive to thiobarbituric acid.**

| Group 1 | Patients | | | | | | | | | | |
|---|---|---|---|---|---|---|---|---|---|---|---|
| TBARS T0 | 0.0046 | 0.0041 | 0.0047 | 0.0042 | 0.0043 | 0.005 | 0.0039 | 0.0044 | 0.0047 | 0.0041 | 0.0042 |
| TBARS T12 | 0.0213 | 0.0219 | 0.021 | 0.023 | 0.0199 | 0.0203 | 0.0229 | 0.0225 | 0.022 | 0.019 | 0.024 |
| | | | | | | | | | | | |

| Group 2 | Patients | | | | | | | | | | |
|---|---|---|---|---|---|---|---|---|---|---|---|
| TBARS T0 | 0.067 | 0.0818 | 0.076 | 0.074 | 0.07 | 0.0731 | 0.083 | 0.0681 | 0.076 | | |
| TBARS T12 | 0.047 | 0.0430 | 0.049 | 0.05 | 0.049 | 0.0412 | 0.045 | 0.051 | 0.0509 | | |

Table 3 shows the average of the levels of TBARS measured at time zero (T0) and after 12 months of treatment for groups 1 and 2 of the patients and the average of the values of TBARS in the control group (CTR). At time TO, group 1 showed TBARS values lower than the values of the control group, in contrast to what was found in group 2, in which patients showed TBARS values higher than the control group.

**Table 3. Serum values of TBARS (µM/µgprot.) in the two groups of NASH patients.**

| | Group 1 | Group 1 | Group 2 | Group 2 | CTR |
|---|---|---|---|---|---|
| Parameters | T0 | T12 | T0 | T12 | |
| TBARS | 0.0044±0.0003 | 0.021±0.001 | 0.074±0.006 | 0.047±0.004 | 0.01±0.002 |

The first group of patients (Group 1) in basal conditions showed a value of substances reactive to thiobarbituric acid (TBARS) lower compared to the control values. In this group, after the start of the treatment with the composition of the invention, TBARS levels increased immediately, but these values returned to normal values at the end of the treatment, after 12 months.

The second group (Group 2) showed a very high value of TBARS in basal conditions, compared to the average value of the control. In these patients, the treatment with the composition of the invention significantly reduced the average level of TBARS (p <0.01). Within the group of 20 patients with metabolic syndrome and NASH, two groups of patients were actually present, which differed in the levels of TBARS at the basal condition, group 1 had low values at time 0 and the second on the contrary had high values (compared to the control group of healthy subjects), getting closer to the values of the control group after 12 months of treatment.

The two groups of patients were then further characterized both at time zero and after 12 months of treatment with the composition of the invention (Table 4). All patients in group 1 had a body mass index (BMI), insulin and HOMA index (Homeostasis Model Assessment - an indicator of insulin resistance) which did not change after 12 months of treatment, only the glycaemia in the blood of group 1 decreased compared to basal value (p<0. 05). On the contrary, in group 2, BMI, insulinemia and HOMA index were significantly reduced (p<0.0001) after the treatment, while glycaemia in the blood remained normal after 12 months, as at the time of basal condition (T0).

**Table 4. Average values of metabolic parameters for the two groups of patients at basal condition (T0) and after 12 months of treatment with the composition of the invention (T12).**

| | Group 1 | | Group 2 | |
|---|---|---|---|---|
| **Parameters** | **T0** | **T12** | **T0** | **T12** |
| **BMI** | **30±1.86** | **30±1.8** | **28±0,50** | **26±0,50***** |
| **Glycaemia** | **116±10** | **105±8,2*** | **99±2.15** | **99±2** |
| **Insulin** | **18±2.26** | **17±3.77** | **23±4.34** | **14±1.9***** |
| **HOMA** | **5±1** | **4.5±1.07** | **5.97±0.6** | **3.43±0.5***** |

| | | | | |
|---|---|---|---|---|
| * p<0.05, ** p<0.005; *** p<0.0001 | | | | |

Therefore, patients in Group 1 who at the time of basal condition had lower TBARS values as compared to the control group, resulted not to show changes after treatment with the composition of the invention, in contrast to patients in Group 2 having high TBARS values as compared to the controls, who responded to the treatment with silybin in the composition.

The use of the composition of the invention therefore reduced the body mass index, insulin and insulin resistance (HOMA) of the sole Group 2, determining thus a treatment of the metabolic syndrome and NASH only for this group of patients.

### Example 3

### Normalization of the lipidomic profile of Group 2

The 20 patients with metabolic syndrome and non-alcoholic steatohepatitis (NASH), divided into two groups as shown in Example 2 were treated with the composition of the invention for 12 months at a dose of 4 sachets/day.

On the serum of 20 patients with metabolic syndrome and NASH and 20 healthy subjects in the control group, lipidomics analysis was performed by MALDI-TOF mass spectroscopy. Lipidomic species studied in the analysis, corresponding to the main peaks were:
Lyso: lysophosphatidylcholine, which can have different combinations of fatty acids with varying length and saturation bound at position C-1.
lyso PC 16:0: lysophosphatidylcholine with palmitic acid
lyso PC 18:2 lysophosphatidylcholine with linoleic acid
lyso PC 18:1 lysophosphatidylcholine with oleic acid
lyso PC 18:0 lysophosphatidylcholine with stearic acid
SM: sphingomyelin
PC: phosphatidylcholine, which can have different combinations of fatty acids with varying length and saturation bound at positions C-1 and C2.
PC 16:0/18:2 phosphatidylcholine, which has a chain of palmitic acid in position C-1 and a chain of linoleic acid in position C2
PC 16:0/18:1 phosphatidylcholine, which has a chain of palmitic acid in position C-1 and a chain of oleic acid in position C2
PC 18:1/18:2 phosphatidylcholine, which has a chain of oleic acid in position C-1 and a chain of linoleic acid in position C2
PC 18:0/18:2 phosphatidylcholine, which has a chain of stearic acid in position C-1 and a chain of linoleic acid in position C2
PC 18:1/18:1 phosphatidylcholine, which has a chain of oleic acid in position C-1 and a chain of oleic acid in position C2
PC 18:2/18:2 phosphatidylcholine, which has a chain of oleic acid in position C-1 and a chain of linoleic acid in position C2
PC 18:0/18:2 phosphatidylcholine, which has a chain of stearic acid in position C-1 and a chain of linoleic acid in position C2
PC 18:1/20:4 phosphatidylcholine, which has a chain of oleic acid in position C-1 and a chain of arachidonic acid in position C2
PC 18:0/20:4 phosphatidylcholine, which has a chain of stearic acid in position C-1 and a chain of arachidonic acid in position C2
PC 18:0/20:3 phosphatidylcholine, which has a chain of stearic acid in position C-1 and a chain of homo-g-linolenic acid in position C2.

A pool of the patients' serum was then made, i.e. a pool of the serum of patients belonging to group 1 and a pool of the serum of patients in group 2. Also a pool of the serum was made for the subjects belonging to the control group. The pools were collected and analyzed before treatment and after the treatment. For each pool graphs were drafted of MALDI-TOF mass spectrometry of organic extracts obtained from the sera of the patients themselves.

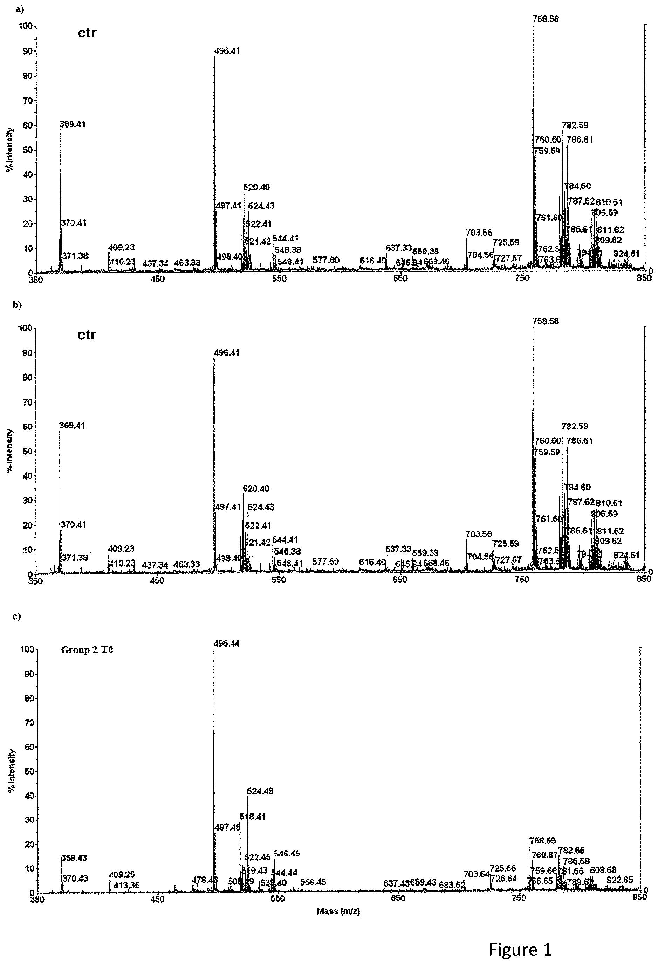

Figure 1 shows the following graphs: spectrogram a. reports the lipidomic profile of the pool of the normal subjects (control group), the spectrogram b. shows the lipidomic profile of the pool of patients belonging to group 1 and the spectrogram c. shows the lipidomic profile of the pool of patients belonging to group 2 (at time 0).

As it is evident from Figure 1, both patients of Group 1 and Group 2 had a lipidic profile different from the profile of the control group. Specifically, all tested species were present in the serum of patients in amounts lower than the amounts of the control subjects (the experiments were repeated three times).

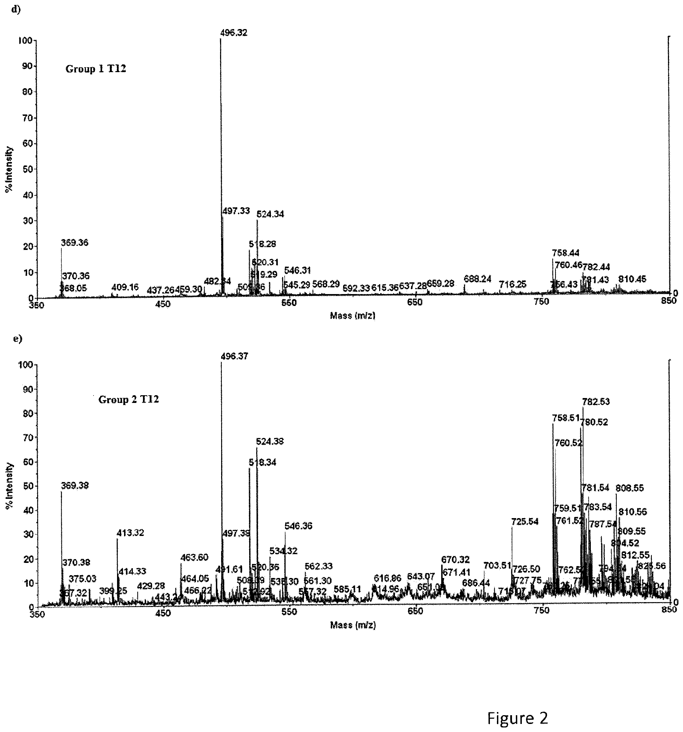

The two groups of patients were then subjected to a treatment with the composition of the invention. After 12 months of treatment with 4 sachets/day, the pools of patient sera, for which the mass spectrum was taken again, were analyzed. Figure 2 shows the mass spectrum of the pool of the same patients in group 1 and group 2 of Figure 1, but after 12 months of treatment with silybin.

As it is evident from Figure 2, the spectrum of the pool of patients in Group 1 showed that the amount of detected lipidic species were still low, as at time zero, and indeed for some species the amount was more decreased.

Conversely, lipidomic profile in Group 2 at T12 (shown in Figure e)) resulted more similar to that one of the control group, thus demonstrating that a normalization had occurred for the lipidomic profile, as tested after the treatment with the composition of the invention.

The intensity of the values from the mass spectrometry was then normalized, and the values are shown in Table 5 below:

**Table 5**

| | **Identity** | **CTR** | **Group 1** | | **Group 2** | |
|---|---|---|---|---|---|---|
| **m/z (MH⁺)** | | | **T₀** | **T₁₂** | **T₀** | **T₁₂** |
| **496,36** | **lyso PC 16:0** | **100** | **100** | **100** | **100** | **100** |
| **369,37** | **FC (-H₂O)(H⁺)** | **68** | **35** | **16** | **15** | **41** |
| **520,4** | **lyso PC 18:2** | **37** | **12** | **2** | **11** | **9** |
| **522,41** | **lyso PC 18:1** | **30** | **18** | **17** | **17** | **15** |
| **524,37** | **lyso PC 18:0** | **31** | **32** | **30** | **30** | **59** |
| **703,5** | **SM 16:0** | **20** | **5** | **1** | **8** | **18** |
| **758,65** | **PC 16:0/18:2** | **104** | **21** | **8** | **19** | **64** |
| **760,51** | **PC 16:0/18:1 (H⁺)** | **42** | **24** | **13** | **8** | **27** |
| **784,66** | **PC 18:1/18:2** | **43** | **13** | **5** | **9** | **34** |
| **786,53 ^{#}** | **PC 18:0/18:2 (H⁺)/ PC 18:1/18:1 (H⁺)** | **40** | **9** | **4** | **6** | **18** |
| **804,52** | **PC 18:2/18:2 (Na⁺)** | **9** | **2** | **1** | **2** | **10** |
| **808,55 ^{#}** | **PC 18:0/18:2 (Na⁺)/ PC 18:1/20:4 (H⁺)** | **19** | **3** | **9** | **5** | **19** |
| **810,55** | **PC 18:0/20:4** | **25** | **5** | **3** | **5** | **18** |
| **812,62** | **PC 18:0/20:3** | **12** | **5** | **22** | **3** | **16** |

| | | | | | | |
|---|---|---|---|---|---|---|
| **# Identification is not unique, molecular weight can be attributed to two possible identities** **FC= free cholesterol** **lyso PC = lysophosphatidylcholine** **PC= phosphatidylcholine** **SM= sphingomyelin** | | | | | | |

Therefore, normalized data confirmed what could be seen from the figures. In NASH patients of group 2, increased circulating levels of free cholesterol, sphingomyelin and phosphatidylcholine could be found after 12 months of treatment. Intensities of these species were low in basal conditions, and returned to control values after the treatment with the composition including silybin. Furthermore, the lipidomic analysis data show that in the group of patients with high TBARS values (Group 2) treatment with the composition including silybin induced an improvement in the ratio phosphatidylcholine/arachidonic acid. As it is well known, sphingomyelin is synthesized in the lumen of the Golgi apparatus, and is then transported to the cell membrane level by specific carriers. Sphingomyelin has a high affinity for cholesterol, with which it binds at the cell membrane level. Without being bound to any theory, based on the above data, the inventors of the present invention assume therefore that the treatment according to the invention catalyzes the release of free circulating sphingomyelin and cholesterol, through an increase in the lyso-phosphatidylcholine ratio, which has a "detergent-like" action on the membranes.

In order to reduce the experimental error, lipid extraction and analysis of lipidomic profile with mass spectrometry were carried out several times for the pool of patients of group 1, group 2, and the control group, thus obtaining results which could be superimposed to those reported above, thus confirming that the lipidomic profile of the patients in group 2 was normalized.

Therefore, the results obtained from the lipidomic analysis, when added to the reductions in the insulinemia and insulin resistance (see Table 3 of Example 2), show that only for patients with metabolic syndrome and NASH of Group 2, the composition including silybin interfered with the cell intermediate metabolism concerning the oxidation of lipids, decreasing the oxidative stress typical of the metabolic syndrome and NASH, and, therefore, having the effects of the treatment on the same only for this specific group of patients.

## Claims

1. A composition for use in the treatment of metabolic oxidative alterations in non-alcoholic steatohepatitis (NASH), wherein said composition comprises silybin and the use is in the treatment of patients having an altered lipidomic profile and wherein the patients having the altered lipidomic profile show an high TBARS value in TBARS test with respect to healthy subjects, wherein said healthy subjects show a TBARS value of 0.01±0.002 µm/µg prot

2. The composition for use according to claim 1, wherein the treatment consists in the normalization of the lipidomic profile of patients having an altered lipidomic profile.

3. The composition for use according claim 1 or claim 2, wherein the composition includes silybin complexed with phosphatidylcholine.

4. The composition for use according to any of claims 1 to 3, wherein silybin is in the form of an extract of *Silybum marianum,* i.e. Milk thistle.

5. The composition for use according to claim 4, wherein silybin titre of the Milk thistle extract is in the range of 20 to 90%, more preferably 30 to 70%.

6. The composition for use according to claim 5, wherein the composition of the invention includes an extract of Milk thistle with a silybin titre of 33%.

7. The composition for use according to any of claims 1 to 6, wherein the composition includes vitamin E.

8. The composition for use according to any of claims 1 to 7, wherein patients having an altered lipidomic profile after the treatment have values of BMI, HOMA and insulin lower than the basal condition.

## Patentansprüche

1. Zusammensetzung zur Verwendung bei der Behandlung von metabolischen, oxidativen Änderungen bei Nicht-alkoholischen Steatohepatitis (NASH), wobei die Zusammensetzung Silybin aufweist und die Verwendung in der Behandlung von Patienten mit einem veränderten lipidomischen Profil besteht und die Patienten mit verändertem lipidomischen Profil in TBARS-Tests gegenüber Gesunden einen hohen TBARS-Wert zeigen und die Gesunden einen TBARS-Wert von 0,01 +/- 0,002 µm/µg prot zeigen.

2. Zusammensetzung zur Verwendung gemäß Anspruch 1, wobei die Behandlung in der Normalisierung des lipidomischen Profils der Patienten mit verändertem lipidomischen Profil besteht.

3. Zusammensetzung zur Verwendung gemäß Anspruch 1 oder 2, wobei die Zusammensetzung mit Phosphatidylcholin komplexiertes Silybin aufweist.

4. Zusammensetzung zur Verwendung gemäß einem der Ansprüche 1 bis 3, wobei das Silybin in Form eines Extrakts von *Silybum marianum* , z.B. Mariendistel, vorliegt.

5. Zusammensetzung zur Verwendung gemäß Anspruch 4, wobei Silybintiter des Mariendistel-Extrakts im Bereich von 20 - 90% vorliegt, bevorzugt 30 - 70%.

6. Zusammensetzung zur Verwendung gemäß Anspruch 5, wobei die Zusammensetzung einen Mariendistel-Extrakt mit einem Silybintiter von 33% aufweist.

7. Zusammensetzung zur Verwendung gemäß einem der Ansprüche 1 bis 6, wobei die Zusammensetzung Vitamin E aufweist.

8. Zusammensetzung zur Verwendung gemäß einem der Ansprüche 1 bis 7, wobei die Patienten mit verändertem lipidomischen Profil nach der Behandlung BMI-, HOMA- und Insulinwerte aufweisen, die niedriger sind als der Basalzustand.

## Revendications

1. Composition pour une utilisation dans le traitement d'altérations oxydatives métaboliques dans la Stéatohépatite non alcoolique (NASH), dans laquelle ladite composition comprend la silibinine et l'utilisation est dans le traitement de patients ayant un profil lipidomique altéré et dans laquelle les patients ayant le profil lipidomique altéré présentent une valeur TBARS élevée dans le test TBARS par rapport à des sujets sains, dans laquelle lesdits sujets sains présentent une valeur TBARS de 0,01 ± 0,002 µm/µg prot.

2. Composition pour une utilisation selon la revendication 1, dans laquelle le traitement consiste en la normalisation du profil lipidomique des patients ayant un profil lipidomique altéré.

3. Composition pour une utilisation selon la revendication 1 ou la revendication 2, dans laquelle la composition comporte la silibinine complexée avec la phosphatidylcholine.

4. Composition pour une utilisation selon l'une quelconque des revendications 1 à 3, dans laquelle la silibinine est sous la forme d'un extrait de *Silybum marianum,* c'est-à-dire de chardon-Marie.

5. Composition pour une utilisation selon la revendication 4, dans laquelle le titre de silibinine de l'extrait de chardon-Marie est dans la plage de 20 à 90 %, de manière davantage préférée 30 à 70 %.

6. Composition pour une utilisation selon la revendication 5, dans laquelle la composition de l'invention comporte un extrait de chardon-Marie avec un titre de silibinine de 33 %.

7. Composition pour une utilisation selon l'une quelconque des revendications 1 à 6, dans laquelle la composition comporte de la vitamine E.

8. Composition pour une utilisation selon l'une quelconque des revendications 1 à 7, dans laquelle des patients ayant un profil lipidomique altéré après le traitement présentent des valeurs d'IMC, de HOMA et d'insuline inferieures à la condition de base.
